# EUROPEAN PATENT APPLICATION

(11) **EP 2 229 976 A1**
(43) Date of publication of application: **22.09.2010**
(21) Application number: 10150869.5
(22) Date of filing: 15.01.2010
(51) Int. Cl.: A61M 31/00, A61K 9/00, A61F 7/02, A61F 7/03

(54) **Skin patch laminate body**

(30) Priority: 18.03.2009 JP 2009066836
(71) Applicant: OKAMOTO INDUSTRIES, INC., Bunkyo-ku Tokyo 113-8710 (JP)
(72) Inventor: Uchiyama, Hitoshi, Ryuugasaki-shi, Ibaraki-ken (JP)
(74) Representative: Hess, Peter K. G.

(57) **Abstract**

A skin patch laminate body (1) is composed of a heat imparting body (10) and a skin patch body (20) having a skin patch functional layer. In the heat imparting body (10), a flat exothermic agent accommodating layer (12) for accommodating an exothermic agent (11) is formed, and a second adhesive layer (14) is formed on a lower surface of the exothermic agent accommodating layer (12). The skin patch body (20) has a base layer (21) for functioning as a substrate of the skin patch body (20), and a skin patch functional layer (22) on a lower surface of the base layer (21). A first adhesive layer (23) for adhering directly to the skin is formed on a lower surface of the skin patch functional layer (22). The second adhesive layer (14) of the heat imparting body (10) is affixed to an upper surface of the base layer (21) of the skin patch body (20) so that the base layer (21) and the heat imparting body (10) are integrated with each other, and the first adhesive layer (23) of the skin patch body (20) is affixed to the skin. The adhesive strength between the second adhesive layer (14) and the base layer (21) is less than the adhesive strength between the first adhesive layer (23) and the skin or other surface.

## Description

### [Technological Field]

The invention relates to a laminate body that includes an exothermic body for application to the skin.

### [Background Technology]

Conventionally, in order to apply a transdermal analgesic anti-inflammatory adhesive plaster for external use to the affected area of the skin and warm the same, a technique has been proposed to arrange a disposal body warmer bonded in advance to the opposite side of the drug application surface of the adhesive plaster (see Patent Reference 1).
A technique has also been proposed in which a protrusion for stimulating an acupuncture point is added to a disposable foot warmer and a magnet is furthermore used in the protrusion in order to relieve symptoms of sensitivity to cold, in addition to achieving the warming effects of the disposable foot warmer (see Patent Reference 2).
In order to achieve a synergism of effects between the heating of a body warmer and the heating stimulation of a medicated patch, a configuration of a medicated patch integrated with a disposable body warmer has been proposed, **characterized in that** an adhesive layer is provided to the front or back of the bag of a disposable body warmer formed by sealing an exothermic material in a bag, a Chinese medicine or other medicated salve is spread on a sheet, a peelable sheet is affixed to the surface of the medicated salve to form a medicated patch, and the salve sheet is affixed to the adhesive layer of the disposable body warmer (see Patent Reference 3). For usage of the medicated patch for a long period of time, a technique has also been proposed in which the disposable body warmer is peeled off the patch when the thermal effects of the disposable body warmer are no longer present, and the salve can be further used by affixing a new disposable body warmer to the patch (see paragraph 0009 of Patent Reference 3).

### [Prior Art References]

### [Patent References]

[Patent Reference 1] Japanese Laid-open Patent Publication No. 2008-200450
[Patent Reference 2] Japanese Laid-open Patent Publication No. 2006-26370
[Patent Reference 3] Japanese Laid-open Patent Publication No. H10-201787

### [Disclosure of the Invention]

### [Problems to Be Solved by the Invention]

However, the disposable body warmers described in Patent References 1 and 2 are configured so that a disposable body warmer and the surface to which the medicine is applied are bonded together, and a protuberance or the like is formed on the warmer as such. Thus it is impossible to separate the disposable body warmer from the other portion that is used for a different therapeutic effect. When the thermal effects of the disposable body warmer are no longer present, the portion used for a different therapeutic effect than the disposable body warmer must also be discarded.

In Patent Reference 3 there is described a skin patch that can be reused by peeling the disposable body warmer off from the skin patch when the thermal effects of the disposable body warmer are no longer present, and affixing a new disposable body warmer to the skin patch. But the disposable body warmer cannot be peeled off without peeling from the skin the sheet on which the drug is spread, and therefore the warmer must be peeled away from the sheet after the sheet has been peeled off of the skin. Moreover, since the adhesion strength of the sheet weakens, and the sheet can easily be peeled off from the following affixing, problems occur in the number of times that the exothermic body as the disposable body warmer can be conveniently peeled off while leaving the portion used for a different therapeutic effect on the skin.

Therefore, it is an object of the present invention to provide a skin patch laminate body having a two-layer structure composed of a heat imparting body and a skin patch body, the skin patch laminate body being provided with a heat imparting body that includes an exothermic agent, and a skin patch body that includes a portion that functions by being affixed to the skin, whereby it is possible to conveniently peel off only the heat imparting body after the exothermic effects of the heat imparting body are no longer present, and a new heat imparting body can be reattached on the skin patch body remaining on the skin.

### [Means Used to Solve the Above-Mentioned Problems]

In order to solve the problems above, it is a primary object of the present invention to provide a skin patch laminate body comprising a skin patch body having a base layer and a first adhesive layer for adhering directly to skin, the first adhesive layer being formed on one side of the base layer; and a heat imparting body having an exothermic agent accommodating layer and a second adhesive layer for adhering to the other side of the base layer, the second adhesive layer being formed on one side of the exothermic agent accommodating layer; wherein the adhesive strength between the second adhesive layer and the base layer is less than the adhesive strength between the first adhesive layer and the skin.

It is an object of the present invention to provide a skin patch laminate body, wherein the skin patch body is equipped with a skin patch functional layer that is made to function on being affixed to the skin.

Another object of the present invention is to provide a skin patch laminate body, wherein the skin patch functional layer is a adhesive medication layer.

A further object of the present invention is to provide a skin patch laminate body, wherein the adhesive strength of the second adhesive layer is 0.2 Newtons/25 mm or greater, and the adhesive strength between the first adhesive layer and the skin is at least 0.8 Newtons/25 mm greater than the adhesive strength between the second adhesive layer and the base layer, the adhesive strength being measured by JIS Z0237 (-2000) (adhesive tape/ adhesive sheet testing method).

A still further object of the present invention is to provide a heat imparting body used in the skin patch laminate body described above, the heat imparting body characterized in comprising a peelable sheet that can be peeled on a side of the second adhesive layer that faces the base layer.

### [Effect of the Invention]

According to the present invention, even after the thermal effects of the heat imparting body have diminished, the heat imparting body can be peeled off while the skin patch body that includes the portion that functions by being affixed to the skin remains affixed to the skin. Thus there is no longer a need for repeated applications to the skin.
After the heat imparting body has been peeled off, a new heat imparting body is reaffixed to provide continuous thermal effects of the heat imparting body. The skin patch laminate body can thereby demonstrate a synergism of the effects of the skin patch body and the thermal effects of the heat imparting body.
A skin patch laminate body having excellent usability can be provided, whereby feeling of chill given when the skin patch body is affixed to the skin can be reduced by the thermal effects of the heat imparting body.

### [Brief Description of the Drawings]

FIG. 1 is a sectional view of a skin patch laminate body to show a basic structure thereof according to an aspect of the present invention;
FIG. 2 is a sectional view of a skin patch laminate body according to a first embodiment of the present invention; and
FIG. 3 is a sectional view of a replacement heat imparting body according to another aspect of the first embodiment of the present invention.

### [Description of Embodiments]

Embodiments of the present invention will next be described with reference to the drawings.

### [Basic structure]

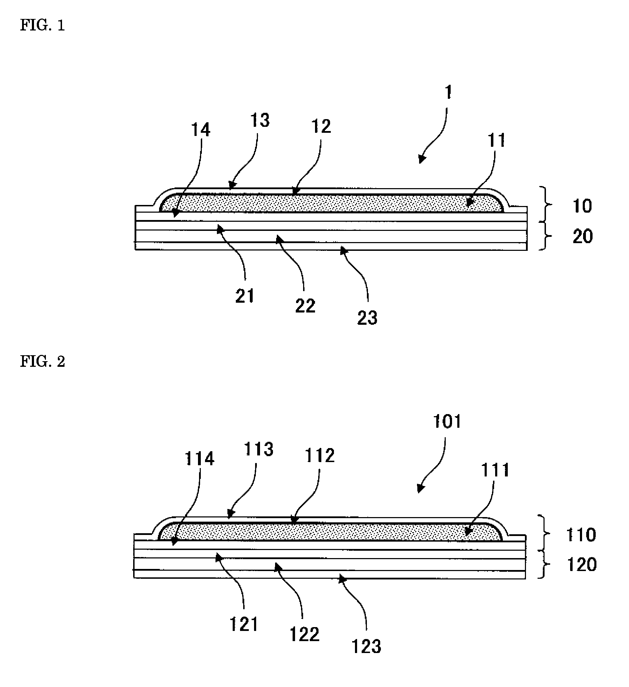

As shown in FIG. 1, a skin patch laminate body 1 includes a heat imparting body 10 and a skin patch body 20 that is equipped with a skin patch functional layer.
The heat imparting body 10 has a flat exothermic agent accommodating layer 12 for accommodating an exothermic agent 11, and a second adhesive layer 14 formed on a lower surface of the exothermic agent accommodating layer 12. The heat imparting body 10 also has, on an upper surface of the exothermic agent accommodating layer 12, a ventilation surface 13 composed of an air permeable film formed for exposing the exothermic agent 11 to the air for oxidative heat generation in the exothermic agent 11.
The skin patch body 20 has a base layer 21 for functioning as a substrate of the skin patch body 20, and a skin patch functional layer 22 that functions by being affixed to the skin, the skin patch functional layer 22 being formed on a lower surface of the base layer 21 and having a medication that includes medicinal properties, a therapeutic solid, or the like. The skin patch body 20 also has a first adhesive layer 23 for adhering directly to the skin formed on the lower surface of the skin patch functional layer 22.
The second adhesive layer 14 of the heat imparting body 10 is affixed to an upper surface of the base layer 21 of the skin patch body 20 so that the base layer 21 and the heat imparting body 10 are integrated with each other, and the first adhesive layer 23 of the skin patch body 20 is affixed to the skin. In this case, the adhesive strength between the second adhesive layer 14 and the base layer 21 is less than the adhesive strength between the first adhesive layer 23 and the skin or other surface. As a result, when the thermal effects of the heat imparting body 10 are no more present, it is easy to peel off only the heat imparting body 10.
Embodiments according to the present invention will be described in further detail below.

### [First embodiment]

As shown in FIG. 2, a skin patch laminate body 101 includes a disposable body warmer 110 as a heat imparting body, and a medicated patch 120 in which a parcutaneous absorption agent or the like having adhesive properties is used as a medication.
The disposable body warmer 110 is provided with an exothermic agent 111 that is sealed inside a flat exothermic agent accommodating layer 112, a ventilation surface 113 composed of an air-permeable film on a upper surface of the exothermic agent accommodating layer 112, and a second adhesive layer 114 on a lower surface of the exothermic agent accommodating layer 112. The exothermic agent 111 is a powder mixture in which iron powder, water, vermiculite, active carbon, salts, and the like are mixed together, and reacts with oxygen to generate heat. The exothermic agent used may be formed by forming the powder mixture into a sheet, or retaining the powder mixture on a sheet. At least a portion of the ventilation surface 113 is air-permeable so as to supply oxygen to the exothermic agent 111 inside; specifically, numerous ventilation holes (not shown) are formed therein. The release of heat can be adjusted by airflow of the ventilation surface 113.

The medicated patch 120 is provided with a base layer 121 composed of a woven cloth, a nonwoven cloth, or other flexible materials; and an adhesive medication layer 122 of an adhesive composition that includes the medicinal properties of a transdermal analgesic, a poultice, or the like on the lower surface of the base layer 121. The adhesive medication layer 122 is composed of patch application such as a transdermal analgesic anti-inflammatory adhesive plaster, a Chinese herb plaster, an ointment, a poultice, a blood circulation promoter, or other topical medications, which may be any medicine that can be applied to the skin to cure shoulder stiffness, lower back pain, muscle pain, and the like. A skin care agent whereby natural effects are enhanced by thermal effects may also be included. For example, using hair removal cream, pores are opened by the thermal effects, thereby effectively removing hairs. The base layer 121 may be a polymer compound such as polyurethane or the like. An acrylic adhesive is preferred for the adhesive used in the second adhesive layer 114.

The second adhesive layer 114 of the disposable body warmer 110 is affixed to the upper surface of the base layer 121 of the medicated patch 120 so that the second adhesive layer 114 and the base layer 121 are integrated with each other, and the adhesive medication layer 122 of the medicated patch 120 is affixed to the skin.
(The disposable body warmer 110 may, of course, be affixed on the base layer 121 after the medicated patch 120 is affixed to the skin.)
The adhesive strength between the second adhesive layer 114 and the base layer 121 is less than the adhesive strength between the adhesive medication layer 122 and the skin, as described above. On the other hand it would be inconvenient if the adhesive strength of the second adhesive layer were too low to prevent peeling off during use. The adhesive strength must be at least 0.2 Newtons/25 mm as measured by JIS (Japanese Industrial Standard) Z0237 ( 2000) (adhesive tape/adhesive sheet testing method) in order to prevent the disposable body warmer 110 from peeling off during use. In this case, the difference in adhesive strength must be 0.8 Newtons/25 mm so as to easily peel off only the disposable body warmer 110.

### (Test Example 1)

The adhesive strength of the adhesive medication layer 122 of the medicated patch 120, and the adhesive strength of the second adhesive layer 114 of the disposable body warmer 110 were measured by JIS Z0237 (-2000) (adhesive tape/adhesive sheet testing method) to determine the difference in adhesive strengths necessary to easily peel off the disposable body warmer 110 with the medicated patch 120 affixed to the skin. As a result, it was concluded that the second adhesive layer should preferably be formed so that the adhesive strength thereof varies in accordance with the adhesive strength of the first adhesive layer, as shown in Table 1. Specifically, since the adhesive strength of the second adhesive layer must be at least 0.2 Newtons/25 mm, as described above, the adhesive strength of the first adhesive layer must be 1.0 Newton/25 mm or greater.
However, since this is a minimum value for preventing peel-off during use. When the adhesive strength of the first adhesive layer is greater, e.g., 1.7 Newtons/25 mm, the adhesive strength of the second adhesive layer can be increased to 0.3 Newton/25 mm. Thus, since the disposable body warmer 110 is further prevented from falling off, and the difference in adhesive strength is increased, the disposable body warmer 110 can be more easily peeled off.
Furthermore, when the adhesive strength of the first adhesive layer is further increased, e.g., to 3.0 Newtons/25 mm, the adhesive strength of the second adhesive layer can also be increased, e.g., to 0.4 Newton/25 mm. In this case, the disposable body warmer 110 is further prevented from falling off, and the difference between the adhesive strengths is increased, and the disposable body warmer 110 can therefore be even more easily peeled off.
The measured values herein were obtained by conducting adhesive strength tests according to the 180-degree peel method with respect to a 10.3.2 back surface in accordance with JIS Z0237 ( 2000) (adhesive tape/ adhesive sheet testing method). Specifically, a piece having the same dimensions as a test sample of an adhesive sheet for a disposable body warmer was taken from an adhesive sheet of a first adhesive layer, the piece was pressed onto an SUS plate, and both ends thereof were folded around and affixed to a back surface of the SUS plate. An adhesive sheet for a disposable body warmer was then pressed onto a back surface thereof by the same method as 10.3.1, and then measured in the same manner as 10.3.1.

**[Table 1]**

| | first adhesive layer (P1) | second adhesive layer (P2) | Difference (P1 - P2) |
|---|---|---|---|
| Minimum | 1.0 | 0.2 | 0.8 |
| Favorable | 1.7 | 0.3 | 1.4 |
| More favorable | 3.0 | 0.4 | 2.6 |

| | | | |
|---|---|---|---|
| (Unit: N/25mm) | | | |

Preferably, a peelable sheet 123 that can be peeled off may be provided to a lower surface of the adhesive medication layer 122, and the peelable sheet 123 may be peeled off to expose the adhesive medication layer 122 when the adhesive medication layer 122 is affixed to the skin.

A shape of the flat skin patch laminate body 101 may be circular, elliptical, or any substantially polygonal shape. When a substantially polygonal shape is adopted, corners thereof are preferably rounded rather than angular to prevent unnecessary separation.

Usage methods according to the present embodiment will next be described.
According to the present embodiment, a unused skin patch laminate body 101 is sealed inside a bag so that the exothermic agent 111 does not come in contact with oxygen and generate oxidative heat. Of course, a configuration may also be adopted in which the skin patch laminate body 101 is not placed in a bag, and a sheet is provided for blocking the ventilation surface 113 so that the exothermic agent 111 does not come in contact with oxygen through the ventilation surface 113.
When a sealed bag is used, a user first opens the bag and takes the skin patch laminate body 101 out of the bag. The user then peels the peelable sheet 123 from the skin patch laminate body 101 and affixes the skin patch laminate body 101 to the skin of the affected area. The exothermic agent 111 begins to generate heat as soon as the bag is ruptured and comes into contact with oxygen. Accordingly, thermal effects may appear more rapidly by increasing the number of ventilation holes in the ventilation surface 113 to increase the rate of exposure to oxygen, or by reducing the moisture content of the exothermic agent 111. As a result, since warming has already begun by the time when the skin patch laminate body 101 is affixed to the skin, the user does not experience a feeling of chill, and realizes immediate warmth.
When thermal effects of the exothermic agent 111 are no longer present, the user peels only the disposable body warmer 110 from the medicated patch 120. At this time, since the adhesive strength between the second adhesive layer 114 and the base layer 121 is less than the adhesive strength between the adhesive medication layer 122 and the skin, the medicated patch 120 remains affixed to the skin and does not peel off along with the disposable body warmer 110.

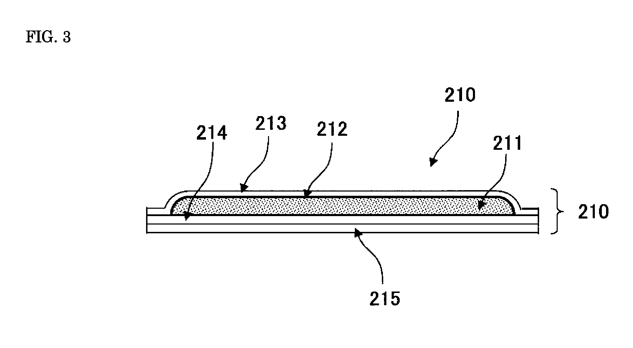

When the user wishes to maintain the thermal effects, the user removes a replacement disposable body warmer 210 shown in FIG. 3 from a bag, peels off a peelable sheet 215 provided to a second adhesive layer 214, and affixes the second adhesive layer 214 to the base layer 121 of the medicated patch 120 affixed to the skin.
Continuous thermal effects of the disposable body warmer (heat imparting body) are provided by re-affixing the new replacement disposable body warmer 210 when the thermal effects of the disposable body warmer are no longer present. It is thereby possible to provide a skin patch laminate body that demonstrates a synergism of the effects of a medicated patch (skin patch body) and the thermal effects of a heat imparting body.

### [Other embodiments]

The present invention is not limited to the first embodiment described above, and the technical scope thereof includes various forms of design modifications in a range that does not depart from the essence of the present invention as described in the claims.

A skin patch body 20 may be provided with a protruding solid body in the skin patch functional layer 22 for stimulating an acupuncture point for such purposes as promoting blood circulation.

A heat imparting body 10 and A skin patch body 20 may each have a different planar shape or size. For example, with the circular skin patch body 20, the heat imparting body 10 may be elliptical.
More specifically, when the radius of the circle and the long axis of the ellipse are substantially equal, the heat imparting body 10 is smaller than the base layer 21 by an amount commensurate with the difference between the radius of the circle and the short axis of the ellipse, resulting in a level difference that provides a starting point for peeling and enables the heat imparting body 10 alone to be easily peeled off. Conversely, the radius of the circle and the short axis of the ellipse may also be made substantially equal.
The heat imparting body 10 is thereby larger than the base layer 21 by an amount commensurate with the difference between the radius of the circle and the long axis of the ellipse, resulting in a pinched portion that provides a starting point for peeling and enables the heat imparting body 10 alone to be easily peeled off.

### [Key to Symbols]

1 skin patch laminate body
10 heat imparting body
11 exothermic agent
12 exothermic agent accommodating layer
13 ventilation surface
14 second adhesive layer
20 skin patch body
21 base layer
22 skin patch functional layer
23 first adhesive layer
101 skin patch laminate body
110 disposable body warmer
111 exothermic agent
112 exothermic agent accommodating layer
113 ventilation surface
114 second adhesive layer
120 medicated patch
121 base layer
122 adhesive medication layer
123 peelable sheet
210 replacement disposable body warmer
211 exothermic agent
212 bag
213 ventilation surface
214 second adhesive layer
215 peelable sheet

## Claims

1. A skin patch laminate body (1, 101) comprising;
a skin patch body (20, 120) having a base layer (21, 121) and a first adhesive layer (23, 123) formed on one side of the base layer (21, 121) for adhering directly to skin, and
a heat imparting body (10, 110) having an exothermic agent accommodating layer (12, 112, 212) and a second adhesive layer (14, 114, 214) formed on one side of the exothermic agent accommodating layer (12, 112, 212) for adhering to the other side of the base layer (21, 121), the second adhesive layer (14, 114, 214), wherein
the adhesive strength between the second adhesive layer (14, 114, 214) and the base layer (21, 121) is less than the adhesive strength between the first adhesive layer (23, 123) and the skin.

2. The skin patch laminate body (1, 101) according to claim 1, wherein the skin patch body (20, 120) comprises a skin patch functional layer (22, 122) that is made to function on being affixed to the skin.

3. The skin patch laminate body (1, 101) according to claim 2, wherein the skin patch functional layer (22) is an adhesive medication layer (122).

4. The skin patch laminate body (1, 101) according to any of claims 1 through 3, wherein the adhesive strength of the second adhesive layer (14, 114, 214) is 0.2 Newtons/25 mm or greater, and the adhesive strength between the first adhesive layer (23, 123) and the skin is at least 0.8 Newtons/25 mm greater than the adhesive strength between the second adhesive layer (14, 114, 214) and the base layer (21, 121), the adhesive strength being measured by JIS Z0237 ( 2000) (adhesive tape/ adhesive sheet testing method).

5. The heat imparting body (10, 110, 210) used in the skin patch laminate body (1, 101) according to claims 1 through 4, comprising a peelable sheet (215) that can be peeled on a side of the second adhesive layer (14, 114, 214) that faces the base layer (21, 121).
